Europäisches Patentamt

European Patent Office

Office européen des brevets

(19)

(11) Numéro de publication: **0 250 288**
**A1**

(12)

# DEMANDE DE BREVET EUROPEEN

(21) Numéro de dépôt: **87401250.3**

(22) Date de dépôt: **04.06.87**

(51) Int. Cl.4: **C 12 N 9/64**
**// A61K37/54**

(30) Priorité: **12.06.86 FR 8608479**

(43) Date de publication de la demande:
**23.12.87 Bulletin 87/52**

(84) Etats contractants désignés:
**AT BE CH DE ES FR GB GR IT LI NL SE**

(71) Demandeur: **INSTITUT MERIEUX Société anonyme dite:**
**17, rue Bourgelat**
**F-69002 LYON (FR)**

(72) Inventeur: **Grandgeorge, Michel**
**12 rue du Recret**
**F-69670 Vaugneray (FR)**

(74) Mandataire: **Bernasconi, Jean et al**
**CABINET LEMOINE ET BERNASCONI 13, BLd. des**
**Batignolles**
**F-75008 Paris (FR)**

(54) Procédé de préparation de facteur XIII à partir du placenta.

(57) On prépare directement à partir du placenta un précipité alcoolique riche en facteur XIII et l'on soumet ensuite ce précipité à une purification pour purifier le facteur XIII obtenu.

EP 0 250 288 A1

# 0 250 288

## Description

Procédé de préparation de facteur XIII à partir du placenta.

La présente invention a trait à un procédé de préparation de facteur XIII ou d'une fraction enrichie en facteur XIII à partir du placenta.

Le facteur XIII ou facteur stabilisant de la fibrine joue un rôle essentiel dans la coagulation du sang, en réticulant et consolidant le caillot formé, après avoir été lui-même activé par la thrombine, assurant ansi un arrêt durable de l'hémorragie. Ce facteur a également une action importante dans les mécanismes de cicatrisation en favorisant la prolifération des fibroblastes et la reconstitution du tissu conjonctif lésé.

Le facteur XIII présente un intérêt thérapeutique chez les patients qui souffrent d'un déficit héréditaire ou acquis en facteur XIII et présentent des symptômes hémorragiques. Il peut être également utilisé en chirurgie pour compenser une baisse post-opératoire du taux de facteur XIII sanguin et favoriser une cicatrisation rapide. L'administration de facteur XIII donne des résultats également intéressants dans le traitement de la sclérodermie systémique. Par ailleurs, le facteur XIII peut être utilisé dans les compositions de colle biologique à base de fibrinogène comme facteur de consolidation.

Le placenta représente la source de facteur XIII la plus intéressante du point de vue économique et on connaît déjà plusieurs procédés d'isolation du facteur XIII à partir de cette source.

Ainsi, le brevet US 4.285.933 décrit un procédé de concentration du facteur XIII à partir d'une fraction brute globulinique obtenue après extraction du sang placentaire à l'aide d'une solution saline, et séparée d'une fraction brute riche en albumine par fractionnement au sulfate d'ammonium. L'extraction du facteur XIII à partir de la fraction globulinique s'effectue par précipitation à pH de 6 à 9 en présence de 4 à 9% d'un polymère ou co-polymère alkylène oxyde tel que le Pluronic, le surnageant étant amené à une concentration de 20 à 30% de ce polyol pour précipiter une fraction riche en facteur XIII qui est ensuite traitée par des étapes connues de chromatographie sur colonnes échangeuses d'ions, dialyse et filtration stérilisante.

Un tel procédé présente l'inconvénient de mettre en oeuvre une étape de fractionnement du sang placentaire au sulfate d'ammonium, procédé peu intéressant pour la récupération ultérieure de l'albumine qui constitue une protéine d'intérêt thérapeutique et économique majeur du placenta. En outre, les gamma-globulines, qui constituent un autre composant protéinique d'intérêt majeur, sont perdues en partie.

Le brevet belge 777.135 décrit un procédé consistant également à procéder à une extraction en solution saline du sang contenu dans la matière placentaire, puis à précipiter la fraction riche en facteur XIII à l'aide de lactate de diamino-éthoxy-acridine (rivanol), après quoi le facteur XIII est purifié à partir de ce précipité à l'aide d'un procédé de fractionnement par le sulfate d'ammonium, suivi d'une filtration sur gel et d'une précipitation à l'état d'euglobuline.

Le brevet belge 777.136 décrit un procédé similaire dans lequel le précipité obtenu lors de l'étape de précipitation au rivanol est traité par un procédé de fractionnement usuel à l'alcool, suivi d'une filtration sur gel et d'une chromatographie sur colonne échangeuse d'ions.

Dans ces deux procédés, la récupération ultérieure de l'albumine et des gamma-globulines est également difficile et ne peut pas être complète.

La demande de brevet européen EP-A-O.126.426 décrit un procédé de préparation de facteur XIII par précipitation alcoolique à pH neutre à taux d'alcool élevé, à partir d'un extrait placentaire, d'un bloc contenant les immunoglobulines et le facteur XIII, suivie d'une remise en solution, puis d'une séparation du facteur XIII d'avec les immunoglobulines par l'utilisation d'une résine échangeuse d'anions, telle que la DEAE-cellulose, puis d'une élution du facteur XIII.

Ce procédé nécessite l'utilisation d'une quantité très importante d'échangeurs d'anions, procédé fastidieux et coûteux

La présente invention se propose de remédier à ces inconvénients et de fournir un procédé de préparation de facteur XIII permettant d'assurer une récupération optimale de l'albumine et des gamma-globulines ainsi que d'autres constituants intéressants du placenta.

Un autre objectif de l'invention est de fournir un tel procédé qui puisse être mis en oeuvre de façon particulièrement économique.

Un autre objectif encore de l'invention est de fournir un tel procédé permettant d'améliorer la stabilité du facteur XIII, non seulement pendant les opérations de purification mais également après la fin de ces opérations.

L'invention a pour objet un procédé de préparation de facteur XIII à partir du placenta dans lequel on effectue une extraction du sang placentaire, caractérisé en ce que l'on isole, soit directement à partir de l'extrait placentaire, soit à partir d'un bloc de globulines obtenu à partir dudit extrait, une préparation de facteur XIII sous forme d'un insoluble ou précipité, par séparation dans un milieu alcoolique à pH acide et faible taux d'alcool, notamment l'éthanol.

L'étape de précipitation selon l'invention permet de séparer de manière pratiquement complète le facteur XIII d'avec les immunoglobulines et, s'il y a lieu, d'avec l'albumine.

Ce précipité de facteur XIII peut être, de préférence, soumis à un fractionnement ultérieur pour obtenir un produit administrable de pureté supérieure et éliminer ou diminuer les effets indésirables lors de l'administration à l'homme.

La teneur en facteur XIII de ce précipité est de l'ordre de 40 à 60% du facteur XIII contenu dans le sang

2

placentaire initial.

Dans une première forme de réalisation, on effectue une précipitation alcoolique du sang placentaire selon la méthode de TAYLOR pour précipiter le bloc des globulines, l'albumine restant dans le surnageant, après quoi on reprend ou disperse, dans une solution saline, le bloc de globulines et l'on sépare les gamma-globulines qui se dissolvent d'avec la fraction insoluble résiduelle qui constitue la fraction riche en facteur XIII.

De préférence, dans ce premier mode de mise en oeuvre, après avoir décongelé le placenta broyé à basse température, on extrait le sang placentaire à une température comprise de préférence entre 0 et 10°C à l'aide d'eau ou d'une solution aqueuse contenant éventuellement un sel, de préférence le chlorure de sodium à une concentration de 4 à 10 g/l et éventuellement un alcool, de préférence l'éthanol, à une concentration de 8 à 15%, de préférence 12% vol/vol si l'on utilise un litre de solution extractive pour 1kg de placenta, de sorte que le taux d'alcool final de l'extrait est faible, par exemple de l'ordre de 8%. Après élimination du tissu par pressage, on précipite le bloc de globulines à pH neutre compris entre 6,5 et 7,5 et à basse température entre 0 et -8°C, par exemple -5 ou -6°C, par addition d'alcool jusqu'à un taux de l'ordre de 20 à 30%, de préférence 25% (vol/vol).

Le précipité de globulines est recueilli puis dispersé en solution saline à un pH acide et à basse température et la fraction insoluble résiduelle qui constitue le précipité riche en facteur XIII, est séparée, par exemple par centrifugation. La fraction soluble qui contient les gamma-globulines peut être ultérieurement soumise au fractionnement conventionnel à l'alcool de TAYLOR.

La dispersion du bloc de globuline s'effectue de préférence à un pH acide compris entre 4,5 et 5,5 ou même 6, et de préférence entre 4,6 et 5,4 de préférence 4,8 à une température comprise entre 0 et 10°C, de préférence 0°C et une concentration en chlorure de sodium de 0 à 10 g/l, de préférence 4 g/l et avec un taux d'alcool résiduel de 2 à 8 ou 10%, de préférence 3,5% (vol/vol).

Dans un second mode de mise en oeuvre, on précipite une fraction insoluble directement à partir de l'extrait placentaire débarrassé du tissu en solution alcoolique de 2 à 10%, de préférence 7% (vol/vol) et à température abaissée. Pour cela, après avoir décongelé le placenta broyé à basse température, on extrait le sang placentaire à une température comprise de préférence entre 0 et 10°C, à l'aide d'eau ou d'une solution aqueuse contenant éventuellement un sel, de préférence le chlorure de sodium à une concentration de 4 à 10 g/l et éventuellement un alcool, de préférence l'éthanol, à une concentration de 8 à 15%, de préférence 12%, de sorte que le taux d'alcool final de l'extrait est faible, par exemple de 7% ; on élimine le tissu par pressage et on procède à une étape de clarification en ajustant le pH acide à une valeur de préférence entre 4,5 et 5,5 ou même 6, et de préférence entre 4,6 et 5,4, notamment 5,1, en solution alcoolique à ladite concentration de 2 à 10%, de préférence 7%, l'alcool pouvant, le cas échéant, provenir de l'extraction précité, si elle est alcoolique, à température de préférence entre 0 et 10°C, par exemple 2°C, permettant d'obtenir un précipité que l'on recueille par centrifugation. Ce précipité constitue la fraction riche en facteur XIII. Le surnageant qui contient les gamma-globulines et l'albumine peut être soumis au fractionnement ultérieur habituel de TAYLOR.

Le précipité formant la fraction riche en facteur XIII contient également une quantité notable de lipides, quel que soit le mode de mise en oeuvre de l'invention.

Le précipité riche en facteur XIII peut ensuite être traité par tout procédé usuel de purification du facteur XIII. On le reprend de préférence d'abord en tampon phosphate à pH 7,2 après quoi on le soumet à des opérations de purification usuelles, par exemple au rivanol, au sulfate d'ammonium, puis chromatographie.

Pour augmenter la stabilité du facteur XIII, on peut avantageusement ajouter, au début et/ou pendant les étapes de préparation, un agent chelatant de calcium tel que l'EDTA (sel sodique de l'acide éthylène diamine tétraacétique) ou un citrate.

D'autres avantages et caractéristiques de l'invention apparaîtront à la lecture de la description suivante, faite à titre d'exemple non limitatif.


Exemple 1.

12 tonnes de placenta humain congelé sont broyés et décongelées à une température inférieure à 10°C dans un volume de 12.000 litres de solution aqueuse contenant 6 g/l de chlorure de sodium et 12% d'alcool éthylique. Le pH est proche de la neutralité. Après élimination du tissu par pressage, le bloc de globulines est précipité d'après la méthode de TAYLOR à un pH neutre et à -6°C par addition d'éthanol jusqu'à un taux d'environ 25%. L'albumine reste dans le surnageant. On recueille par centrifugation le précipité de globulines que l'on disperse à pH 4,8 dans environ 10.000 litres d'une solution à 0°C contenant 4 g/l de chlorure de sodium. La fraction restant insoluble, qui est dénommée ci-après précipité B1, est recueillie par centrifugation. Le surnageant qui contient les gamma-globulines est soumis au fractionnement à l'alcool de TAYLOR permettant de récupérer de manière optimale les gamma-globulines.

On procède ensuite à une extraction d'une partie aliquote du précipité B1 par 5 à 10 volumes de tampon phosphate 50 mM à pH 7,2, contenant 2 g/l d'EDTA.

Après centrifugation, on recueille le surnageant qui contient le facteur XIII.

L'activité de ce surnageant en facteur XIII est mesurée à l'aide d'un test de dissolution d'un caillot de fibrine par l'acide monochloracétique. Une unité correspond à l'activité d'un ml du plasma obtenu d'un pool de donneurs.

Le rendement mesuré est de 1.155 unités/kg de placenta avec une activité spécifique de 537 unités/g de protéines (valeurs moyennes obtenues à partir de 11 lots de 12 tonnes de placentas).

0 250 288

Le facteur XIII présent dans cet extrait de précipité B1 représente environ 40% du facteur XIII contenu dans le placenta.

Exemple 2.

On reprend le procédé décrit dans l'exemple 1 en ajoutant de l'EDTA à la solution de 6 g/l de chlorure de sodium contenant 12% d'alcool éthylique dans laquelle on broie et décongèle le placenta de façon à obtenir une concentration de 1 à 2 g/l d'EDTA.

On ajoute également de l'EDTA à 1 à 2 g/l au volume de solution à 4 g/l de chlorure de sodium dans laquelle on disperse le bloc de globulines.

La stabilité de l'extrait phosphate du précipité B1 dans le temps a été déterminée de la façon suivante : l'activité en facteur XIII est mesurée, comme dans l'exemple 1, sur l'extrait fraîchement obtenu, puis à nouveau après une journée de repos à 20°C. La baisse d'activité observée dans ce laps de temps sur l'extrait obtenu dans cet exemple est égale à 21% contre 75% dans l'exemple 1 (valeurs moyennes sur 4 essais).

Exemple 3.

3 tonnes de placenta humain congelé sont broyées et décongelées à une température inférieure à 10°C dans un volume de solution contenant 12% d'alcool éthylique.

Après élimination du tissu par pressage, on procède à une étape de clarification.

Pour cette étape de clarification, on abaisse le pH à 5,1 à une température de 2°C. On recueille la partie insoluble par centrifugation. Cette fraction insoluble est appelée "nouveau précipité B1".

Le surnageant, qui contient les gamma-globulines et l'albumine, est soumis aux étapes de fractionnement de TAYLOR usuelles permettant la préparation de fractions purifiées de gamma-globulines et, en combinaison avec d'autres méthodes connues, d'albumine.

Le "nouveau précipité B1" est extrait par 5 à 10 volumes de tampon phosphate de 50 mM à pH 7,2 contenant de 1 à 2 g/l d'EDTA. On mesure son activité comme dans l'exemple 1. Les résultats sont portés dans le tableau 1 qui relate, plus précisément, les résultats de deux essais A et B.

TABLEAU 1

| STADE | ACTIVITE FACTEUR XIII (U /kg placenta) | |
|---|---|---|
| | ESSAI A | ESSAI B |
| Sang placentaire | 2.160 (100 %) | 3.147 (100 %) |
| Nouveau précipité B$_1$ | 940 ( 43 %) | 1.580 ( 50 %) |

Entre parenthèses : le rendement d'extraction du facteur XIII

Exemple 4.

Un extrait en 10 volumes de tampon phosphate obtenu comme dans l'exemple 2 et appelé ci-après "EXTRAIT B1" est purifié comme suit : Après ajustement du pH à 7,0 avec de l'acide chlorhydrique dilué on ajoute à une température de + 4°C du rivanol en solution à 30 g/l pour obtenir un taux final de 1,5 g/l. Le précipité obtenu est séparé par centrifugation puis extrait à pH 7,2 par 10 volumes d'une solution à 25 g/l de chlorure de sodium et 2 g/l d'EDTA. On sépare l'insoluble par centrifugation et mesure l'activité du surnageant, appelé ci-après "EXTRAIT Rivanol", comme expliqué dans l'exemple 1 pour l'extrait du précipité B1 en tampon phosphate ; les résultats obtenus lors de 3 expériences séparées sont reportés dans le tableau 2.

4

TABLEAU 2

| | EXTRAIT B$_1$ ACTIVITE FACTEUR III | | EXTRAIT RIVANOL ACTIVITE FACTEUR III | |
|---|---|---|---|---|
| | U/Kg placenta | Activité spéc. U/g protéine | U/Kg placenta | Activité spéc. U/g protéine |
| Essai 1 | 2.157 (100 %) | 455 | 1.412 (65 %) | 1.032 |
| Essai 2 | 1.698 (100 %) | 483 | 879 (52 %) | 729 |
| Essai 3 | 1.826 (100 %) | 292 | 1.454 (80 %) | 1.237 |

Entre parenthèse : le rendement de purification

Exemple 5

Un extrait en 10 volumes de tampon phosphate est obtenu comme dans l'exemple 1, c'est-à-dire sans addition d'EDTA à l'extraction du sang placentaire et du bloc des globulines. Il est purifié par le rivanol comme dans l'exemple 4. La stabilité de l'extrait rivanol dans le temps est mesurée comme indiqué dans l'exemple 2 pour l'extrait du précipité B1. La baisse d'activité observée est égale à 78 % contre 15 % en moyenne pour les essais 1, 2 et 3 de l'exemple 4.

**Revendications**

1. Procédé de préparation de facteur XIII à partir du placenta dans lequel on effectue une extraction du sang placentaire, caractérisé en ce que l'on isole, soit directement à partir de l'extrait placentaire obtenu, soit à partir d'un bloc de globulines obtenu à partir dudit extrait, une préparation de facteur XIII sous forme d'un insoluble ou précipité, par séparation dans un milieu alcoolique à pH acide et faible taux d'alcool, notamment l'éthanol.

2. Procédé selon la revendication 1, caractérisé en ce que l'on effectue une précipitation alcoolique du bloc des globulines, après quoi on reprend le bloc de globulines en solution saline et l'on sépare les globulines d'avec la fraction insoluble restante, laquelle constitue la fraction riche en facteur XIII.

3. Procédé selon la revendication 2, caractérisé en ce que l'on reprend le bloc de globulines à pH acide entre 4,5 et 5,5 ou 6, à température comprise entre 0 et 10°C, notamment 0°C et à une concentration en chlorure de sodium de 0 à 10 g/l, notamment 4 g/l avec un taux d'alcool résiduel de 2 à 8 ou 10%, notamment 3,5%.

4. Procédé selon la revendication 3, caractérisé en ce que le pH est compris entre 4,6 et 5,4, notamment 4,8.

5. Procédé selon l'une des revendications 3 et 4, caractérisé en ce que l'on précipite le bloc de globulines à pH neutre, compris entre 6,5 et 7,5, basse température, comprise entre 0 et -8°C notamment - 5°C et addition d'alcool notamment éthanol jusqu'à un taux de 20 % à 30 %, de préférence de 25 % Vol/Vol.

6. Procédé selon la revendication 1, caractérisé en ce que l'on effectue une étape de clarification, à pH acide et en présence d'alcool, du sang placentaire et que l'on récupère le précipité obtenu qui constitue la fraction riche en facteur XIII.

7. Procédé selon la revendication 6, caractérisé en ce que l'étape de clarification s'effectue en solution alcoolique de l'ordre de 2 à 10%, notamment 7 %, à température abaissée comprise entre 0 et 10°C, de préférence + 2°C, à pH acide compris entre 4,5 et 5,5 ou 6.

8. Procédé selon la revendication 7, caractérisé en ce que le pH est compris entre 4,6 et 5,4, notamment 5,1.

9. Procédé selon l'une quelconque des revendications 1 à 8, caractérisé en ce que la fraction riche en facteur XIII est soumise à des étapes de purification supplémentaires, notamment de précipitation au rivanol.

10. Procédé seon l'une quelconque des revendications 1 à 9, caractérisé en ce que l'on ajoute, au début et pendant l'étape de préparation un agent tel que de l'EDTA ou citrate, pour augmenter la stabilité du facteur XIII.

11. Procédé selon l'une quelconque des revendications 1 à 10, caractérisé en ce qu'on extrait le sang placentaire du placenta à une température comprise entre 0 et 10°C à l'aide d'eau ou d'une solution aqueuse, notamment de chlorure de sodium à une concentration entre 4 et 10 g/l.

12. Procédé selon la revendication 11, caractérisé en ce que la solution aqueuse contient de l'alcool, notamment l'éthanol à une concentration de 8 à 15%, notamment 12%.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | EP-A-0 162 426 (BEHRINGWERKE AG)<br>* Page 1, ligne 18 - page 3, ligne 19 * | 1-12 | C 12 N    9/64 //<br>A 61 K   37/54 |
| | --- | | |
| Y,D | EP-A-0 011 739 (THE GREEN CROSS CORP.)<br>* Page 5, ligne 11 - page 7, ligne 22 * | 1-12 | |
| | --- | | |
| Y,D | FR-A-2 119 008 (BEHRINGWERKE AG)<br>* Page 2, ligne 6 - page 5, ligne 24 * | 1-12 | |
| | --- | | |
| Y,D | FR-A-2 119 009 (BEHRINGWERKE AG)<br>* Page 2, ligne 6 - page 4, ligne 32 * | 1-12 | DOMAINES TECHNIQUES RECHERCHES (Int. Cl.4)<br><br>A 61 K |
| | ----- | | |

Le présent rapport de recherche a été établi pour toutes les revendications

| Lieu de la recherche | Date d'achèvement de la recherche | Examinateur |
|---|---|---|
| LA HAYE | 21-09-1987 | REMPP G.L.E. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03 82